(19) Europäisches Patentamt
European Patent Office
Office européen des brevets

(11) Veröffentlichungsnummer: **0 004 895**
**B1**

(12) **EUROPÄISCHE PATENTSCHRIFT**

(45) Veröffentlichungstag der Patentschrift: **01.04.81**

(21) Anmeldenummer: **79100982.2**

(22) Anmeldetag: **31.03.79**

(51) Int. Cl.³: **C 07 D 317/62,**
A 01 N 29/04,
A 01 N 43/30,
C 07 D 317/64

(54) **Dichlorbenzodioxolderivate, Verfahren zu ihrer Herstellung und ihre Verwendung als Synergisten in insektiziden und akariziden Mitteln.**

(30) Priorität: **15.04.78 DE 2816474**

(43) Veröffentlichungstag der Anmeldung:
**31.10.79 Patentblatt 79/22**

(45) Bekanntmachung des Hinweises auf die Patenterteilung:
**01.04.81 Patentblatt 81/13**

(84) Benannte Vertragsstaaten:
**BE CH DE FR GB IT NL SE**

(56) Entgegenhaltungen:
**FR - A - 2 315 850**

(73) Patentinhaber: **BAYER AG**
***Zentralbereich Patente, Marken und Lizenzen***
**D-5090 Leverkusen 1, Bayerwerk (DE)**

(72) Erfinder: **Mues, Volker, Dr.**
**Maréestrasse 61**
**D-5600 Wuppertal 1 (DE)**
Erfinder: **Behrenz, Wolfgang, Dr.**
**Untergründemich 14**
**D-5063 Overath (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 004 895 B1

Dichlorbenzodioxolderivate, Verfahren zu ihrer Herstellung und ihre Verwendung als Synergisten in insektiziden und akariziden Mitteln

Die Erfindung betrifft neue Dichlorbenzodioxolderivate, ein Verfahren zu ihrer Herstellung sowie ihre Verwendung als Synergisten in insektiziden und akariziden Mitteln.

Es ist bereits bekannt, daß die folgenden Wirkstoffe bzw. Wirkstoffgruppen pestizide, insbesondere insektizide und akarizide Eigenschaften besitzen:

A) Carbamate, wie z.B. das 2-iso-Propoxy-phenyl-N-methyl-carbamat, 3,4,5-Trimethyl-phenyl-N-methyl-carbamat, 1-Naphthyl-N-methyl-carbamat, 2,3-Dihydro-2,2-dimethyl-7-benzofuranyl-N-methyl-carbamat, 2-(1,3-Dioxolan(2)yl-phenyl)-N-methyl-carbamat und 2,2-Dimethyl-1,3-benzodioxol(4)yl-N-methyl-carbamat,

B) Carbonsäureester, wie z.B. 2,3,4,5-Tetrahydrophthalimido-methyl-chrysanthemat und (5-Benzyl-3-furyl)-methyl-2,2-dimethyl-3-(2-methyl-propenyl)-cyclopropancarboxylat,

C) Phosphorsäureester, wie z.B. 0,0-Dimethyl-0-(2,2-dichlorvinyl)-phosphorsäureester und

D) Halogenalkane, wie z.B. 1,1,1-Trichlor-2,2-bis-(4-methoxyphenyl)-äthan und 1,1,1-Trichlor-2,2-bis(4-chlorphenyl)-äthan.

In der französischen Patentanmeldung 2 315 850 werden bestimmte Methylendioxybenzolderivate beschrieben, wobei auch auf die Wirksamkeit solcher Verbindungen als Synergisten für Insektizide hingewiesen wird.

Weiterhin sind synergistische Mischungen von Carbamaten, z.B. 2-iso-Propoxy-phenyl-N-methylcarbamat oder von Phosphorsäureestern, z.B. 0,0-Diäthyl-0-(2-iso-propyl-4-methylpyrimidin(6)yl)-thionophosphorsäureester oder von natürlichen oder synthetischen Pyrethroiden mit Piperonyläthern, wie z.B. $\alpha$-(2-(2-Butoxy-äthoxy)-äthoxy)-4,5-methylendioxy-2-propyl-toluol, bekannt (vergleiche Bull. Org. mond. Santé/Bull. Wld. Hlth Org. 1966, 35 691—708; Schrader, G.: Die Entwicklung neuer insektizider Phosphorsäureester 1963, S. 158). Doch ist die Wirksamkeit dieser synergistischen Wirkstoffkombination nicht befriedigend. Eine gewisse praktische Bedeutung hat bisher nur das $\alpha$-(2-(2-Butoxy-äthoxy)-äthoxy)-4,5-methylendioxy-2-propyl-toluol erlangt.

Es wurden nun die neuen Dichlorbenzodioxolderivate der Formel (I)

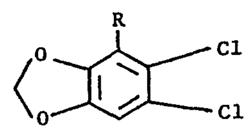

(I)

in welcher

R für Alkyl, Alkenyl, Aralkyl, Aryl, O-Alkyl, O-Alkenyl, O-Aralkyl, oder O-Aryl steht,

gefunden.

Weiter wurde gefunden, daß man die neuen Dichlorbenzodioxolderivate der Formel I erhält, wenn man Benzodioxolderivate der Formel (Ia)

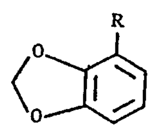

(Ia)

in welcher

R die oben angegebene Bedeutung hat,

mit Sulfurylchlorid, gegebenenfalls in Gegenwart eines Verdünnungsmittels, umsetzt.

Es wurde außerdem gefunden, daß die neuen Dichlorbenzodioxolderivate der Formel I in Kombination mit

A) Carbamaten und/oder

B) Carbonsäureestern, einschließlich der natürlichen sowie synthetischen Pyrethroide, und/oder

C) Phosphorsäureestern und/oder

D) Halogenalkanen

eine besonders hohe insektizide und akarizide Wirkung aufweisen.

Die synergistische Wirkung der Verbindungen der allgemeinen Formel I zeigt sich bevorzugt bei

A) Carbamaten der allgemeinen Formel (II)

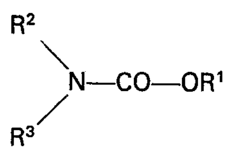

(II)

in welcher

R für Aryl, einen Heterocyclus oder einen Oximrest steht,
$R^2$ für Wasserstoff oder einen Alkylrest mit 1 bis 4 Kohlenstoffatomen steht und
$R^3$ für Alkyl, Alkylcarbonyl mit 1 bis 6 Kohlenstoffatomen im Alkylrest, der gegebenenfalls auch durch Hydroxy oder Methylthio substituiert sein kann, oder den Rest —S—Z steht, wobei
Z für einen gegebenenfalls durch Halogen substituierten aliphatischen Rest mit 1 bis 4 Kohlenstoffatomen, insbesondere $CCl_3$ und $CF_3$ sowie für gegebenenfalls bevorzugt durch Nitril, Halogen, insbesondere Chlor, Methyl, Trihalogenmethyl, Trifluoromethylmercapto oder Nitro substituierten Arylrest, insbesondere Phenyl, oder für Methoxycarbonyl steht.

Besonders bevorzugt sind Carbamate der Formel II, worin
$R^1$ für Phenyl oder Naphthyl steht, die gegebenenfalls substituiert sind durch Alkyl, Alkenyl, Alkoxy, Alkylmercapto oder Alkylthioalkyl mit jeweils 1 bis 6 Kohlenstoffatomen, Dialkylamino, Dialkenylamino mit bis zu 3 Kohlenstoffatomen je Alkyl- bzw. Alkenylteil, Halogen, insbesondere Chlor, Dioxolanyl oder den Rest $—N{=}CH—N(C_{1-4}\text{-Alkyl})_2$ steht.

Weiterhin sind besonders bevorzugt Carbamate der Formel II, worin
$R^1$ für 2,3-Dihydrobenzofuranyl, Benzodioxol, Benzothienyl, Pyrimidinyl oder Pyrazolyl steht, die gegebenenfalls durch Alkylgruppen mit 1 bis 4 Kohlenstoffatomen, insbesondere Methyl, oder durch Dialkylaminogruppen mit 1 bis 4 Kohlenstoffatomen je Alkylteil substituiert sind.

Weiterhin sind besonders bevorzugt Carbamate der allgemeinen Formel II, worin $R^1$ für einen Rest der Formel (IIa)

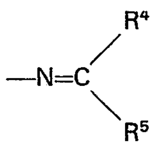

(IIa)

steht, in welcher
$R^4$ und $R^5$ gleich oder verschieden sind und für Alkyl, Cycloalkyl, Alkenyl, Alkinyl, Alkoxy, Alkylmercapto, Alkoxycarbonyl, Carbonylamid, Alkylmercaptoalkyl, mit jeweils bis zu 5 Kohlenstoffatomen, Nitril, Aryl, insbesondere Phenyl, einen gegebenenfalls substituierten heterocyclischen Rest oder für Alkyl, das durch einen heterocyclischen Rest substituiert ist oder gemeinsam einen gegebenenfalls durch $C_{1-4}$-Alkyl substituierten Dioxolanyl- oder Dithiolanylrest stehen.

Besonders erwähnt seien folgende Carbamate der Formel II: 2-Methylphenyl-, 2-Äthylenphenyl-, 2-n-Propylphenyl-, 2-Methoxyphenyl-, 2-Äthoxyphenyl-, 2-iso-Propoxyphenyl-, 4-Methylphenyl-, 4-Äthylphenyl-, 4-n-Propylphenyl-, 4-Methoxyphenyl-, 4-Äthoxyphenyl-, 4-n-Propoxyphenyl-, 3,4,5-Trimethylphenyl-, 1-Naphthyl-, 2,3-Dihydro-2,2-dimethyl-7-benzofuranyl-, 2-(1,3-Dioxolan(2)yl-phenyl)- bzw. 2,2-Dimethyl-1,3-benzodioxol(4)yl-N-methyl-carbamat und die entsprechenden -N-methyl-N-acetyl-, -N-methyl-N-trifluormethylthio, -N-methyl-N-dichlormonofluormethylthio- bzw. -N-methyl-N-dimethylaminothiocarbamate.

Weiter zeigt sich die synergistische Wirkung der Verbindungen der allgemeinen Formel I bevorzugt bei

B) Carbonsäureestern der allgemeinen Formel (III)

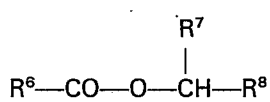

(III)

in welcher
$R^6$ für Alkyl, Aralkyl, Aryl oder Cycloalkyl steht, die gegebenenfalls substituiert sein können,
$R^7$ für Wasserstoff, Alkyl, Halogenalkyl, Alkenyl, Alkinyl, Nitril steht und
$R^8$ für Aryl oder einen Heterocyclus steht, oder gemeinsam mit $R^7$ einen gegebenenfalls substituierten Cyclopentenonring bildet.

Besonders bevorzugt sind Carbonsäureester, in denen $R^6$ für Alkyl mit 1 bis 6 Kohlenstoffatomen, das gegebenenfalls durch gegebenenfalls halogensubstituiertes Phenyl substituiert ist, Cyclopropyl, das gegebenenfalls durch Alkyl, Alkenyl, Halogenalkyl oder Halogenalkenyl mit jeweils bis zu 6 Kohlenstoffatomen substituiert ist, oder für Phenyl, das gegebenenfalls durch Halogen substituiert ist, steht. Bevorzugt sind Carbonsäureester in denen $R^7$ für Wasserstoff, Alkyl mit 1 bis 6 Kohlenstoffatomen, Halogenalkyl mit 1 bis 4 Kohlenstoffatomen und bis zu 4 Halogenatomen, Nitril oder Äthinyl steht.

Weiter sind besonders bevorzugt Carbonsäureester, in denen $R^8$ für Phenyl steht, das gegebenenfalls durch $C_{1-4}$-Alkyl, Halogen, insbesondere Fluor oder Chlor, gegebenenfalls halogen- oder methylsubstituiertes Phenoxy, gegebenenfalls substituiertes Benzyl substituiert ist, ferner für Furanyl, Tetrahydrophthalimido, Benzodioxol, die gegebenenfalls durch Halogen, insbesondere Chlor, Alkyl oder Alkenyl mit bis zu 4-Kohlenstoffatomen oder Benzyl substituiert sind, steht, und ferner für Cyclopentonon steht, das gegebenenfalls durch $C_{1-4}$-Alkyl, Furfuryl, $C_{1-5}$-Alkenyl substituiert ist.

Im einzelnen seien genannt:

Essigsäure-(1-(3,4-dichlorphenyl)-2,2,2-trichloräthyl)-ester, 2,3,4,5-Tetrahydrophthalimidomethylchrysanthemat und (5-Benzyl-3-furyl)-methyl-2,2-dimethyl-3-(2-methyl-propenyl)-cyclopropancarboxylat. Weiter sind besonders bevorzugt die natürlich vorkommenden Pyrethroide.

Weiter zeigt sich die synergistische Wirkung der Verbindungen der allgemeinen Formel I bevorzugt bei

C) Phosphorsäureestern der allgemeinen Formel IV

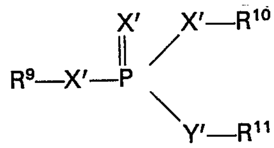

(IV)

in welcher

X' unabhängig voneinander für O oder S steht und

Y' für O, S, —NH— oder für eine direkte Bindung zwischen dem zentralen P-Atom und dem $R^{11}$ steht und

$R^9$ und

$R^{10}$ gleich oder verschieden sind und für Alkyl oder Aryl stehen,

$R^{11}$ für Alkyl, Aryl, Heteroaryl, Aralkyl, Alkenyl, Dioxanyl, oder einen Oximrest oder für den gleichen Rest steht, an den es gebunden ist.

Besonders bevorzugt sind Phosphorsäureester, in denen

$R^9$

und

$R^{10}$ gleich oder verschieden sind und für $C_{1-4}$-Alkyl oder Phenyl stehen,

$R^{11}$ für Alkyl mit 1 bis 4 Kohlenstoffatomen steht, das gegebenenfalls durch Halogen, Hydroxyl, Nitril, gegebenenfalls halogensubstituiertes Phenyl, Carbonylamid, Sulfonylalkyl, Sulfoxyalkyl, Carbonylalkyl, Alkoxy, Alkylmercapto, Alkoxycarbonyl, substituiert ist, für Alkenyl mit bis zu 4 Kohlenstoffatomen, das gegebenenfalls durch Halogen, gegebenenfalls halogensubstituiertes Phenyl oder Alkoxycarbonyl substituiert ist, oder für den Oximrest der allgemeinen Formel

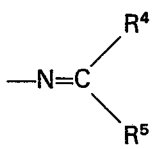

(IIa)

wobei $R^4$ und $R^5$ die oben angegebene Bedeutung besitzen, insbesondere jedoch für Cyan oder Phenyl stehen,

$R^{11}$ steht ferner für Dioxanyl, das durch denselben Rest substituiert ist, an den $R^{11}$ gebunden ist, oder $R^{11}$ steht für den gleichen Rest, an den er gebunden ist, oder $R^{11}$ steht für Phenyl, das gegebenenfalls durch Methyl, Nitro, Nitril, Halogen, Methylthio substituiert ist; $R^{11}$ steht außerdem besonders bevorzugt für gegebenenfalls durch $C_{1-4}$-Alkyl, Halogen substituierte Heteroaromaten, wie Pyridin, Chinolin, Chinoxalin, Pyrimidin, Diazinon, 1,2,4-triazin.

Im einzelnen seien genannt:

O,O-Dimethyl- bzw. O,O-Diäthyl-O-(2,2-dichlor-bzw. 2,2-dibromvinyl)-phosphorsäureester,
O,O-Diäthyl-O-(4-nitro-phenyl)-thionophosphorsäureester,
O,O-Dimethyl-O-(3-methyl-4-methylthio)-thionophosphorsäureester,
O,O-Dimethyl-O-(3-methyl-4-nitro)-thionophosphorsäureester,
O-Äthyl-S-n-propyl-O-(2,4-dichlorphenyl)-thionophosphorsäureester,
O-Äthyl-S-n-propyl-O-(4-methylthio-phenyl)-thionophosphorsäureester,
O,O-Dimethyl-S-(4-oxo-1,2,3-benzotriazin(3)yl-methyl)-thionothiolphosphorsäureester,
O-Methyl-O-(2-iso-propyl-6-methoxy-pyrimidin(4)yl)-thionomethanphosphonsäureester,
O,O-Diäthyl-O-(2-iso-propyl-6-methyl-pyrimidin(4)yl)-thionophosphorsäureester,
O,O-Diäthyl-O-(3-chlor-4-methyl-cumarin(7)yl)-thionophosphorsäureester,
O,O-Dimethyl-2,2,2-trichlor-1-hydroxy-äthan-phosphonsäureester,
O,O-Dimethyl-S-(methylcarbamoylmethyl)-thionophosphorsäureester.

Weiter zeigt sich die synergistische Wirkung der Verbindungen der allgemeinen Formel I bevorzugt bei

D) Halogenalkanen der Formel

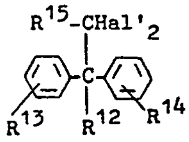

(V)

in welcher

Hal' für Chlor oder Brom und

$R^{12}$ für Wasserstoff oder Hydroxyl stehen,

$R^{13}$

und

$R^{14}$ gleich oder verschieden sind und für Halogen, Alkyl oder Alkoxy und

$R^{15}$ für Wasserstoff oder Halogen stehen.

Besonders bevorzugt sind Halogenalkane, in denen

$R^{12}$ Wasserstoff oder Hydroxyl bedeutet,

$R^{13}$

und

$R^{14}$ für gleiches Halogen, Alkyl bzw. Alkoxy mit 1 bis 4 Kohlenstoffatomen je Alkyl- bzw. Alkoxyrest stehen und

$R^{15}$ Halogen bedeutet.

Im einzelnen seien genannt:

1,1,1-Trichlor-2,2-bis(4-chlor- bzw. 4-methoxyphenyl)-äthan-, 1,1-Trichlor-2-hydroxy-2,2-bis(4-chlorphenyl)-äthan und 1,1-Dichlor-2,2-bis(4-äthylphenyl)-äthan.

Überraschenderweise ist die insektizide und/oder akarizide Wirkung der erfindungsgemäßen Wirkstoffkombinationen wesentlich höher als die Wirkung der Einzelkomponenten bzw. die Summe der Wirkungen der Einzelkomponenten. Sie ist ferner wesentlich höher als die Wirkung der bereits bekannten Wirkstoffkombination aus 2-iso-Propoxyphenyl-N-methyl-carbamat und Piperonylbutoxyd. Außerdem zeigen die erfindungsgemäß verwendbaren Benzodioxol-Derivate ausgezeichnete synergistische Wirksamkeit nicht nur bei einer Wirkstoffklasse, sondern bei Wirkstoffen aus den verschiedensten chemischen Stoffgruppen.

Somit stellen die erfindungsgemäßen Benzodioxol-Derivate und die sie enthaltenden synergistischen Mischungen eine wertvolle Bereicherung der Technik dar.

Die für die erfindungsgemäße Kombination zu verwendenden Synergisten sind durch die Formel I allgemein definiert. Vorzugsweise steht darin jedoch

R für geradkettiges oder verzweigtes Alkyl mit 1 bis 6, insbesondere 1 bis 4 Kohlenstoffatomen je Alkylrest,

für geradkettiges oder verzweigtes Alkenyl mit 2 bis 6, insbesondere 2 bis 4 Kohlenstoffatomen je Alkenylrest,

für Benzyl und Phenyl,

für geradkettiges oder verzweigtes O-Alkyl mit 1 bis 6, insbesondere 1 bis 4 Kohlenstoffatomen je Alkylrest,

für geradkettiges oder verzweigtes O-Alkenyl mit 2 bis 6, insbesondere 2 bis 4 Kohlenstoffatomen je Alkenylrest,

für Benzyloxy und Phenoxy.

Als Beispiele seien im einzelnen genannt:

3-Methyl-, 3-Äthyl-, 3-n-Propyl, 3-iso-Propyl-, 3-n-Butyl-, 3-iso-Butyl-, 3-sek.-Butyl-, 3-tert.-Butyl-, 3-Propen(2)yl-, 3-(2-Methyl-propen(2)yl)-, 3-Buten(2)-yl-, 3-Benzyl- und 3-Phenyl- 4,5-dichlor-1,2-methylendioxybenzol sowie 3-Methoxy-, 3-Athoxy-, 3-n-Propoxy-, 3-iso-Propoxy-, 3-n-Butoxy-, 3-iso-Butoxy-, 3-sek.-Butoxy-, 3-tert.-Butoxy-, 3-Propen(2)oxy-, 3-(2-Methyl-propen(2)oxy)-, 3-Buten(2)oxy-, 3-Benzyloxy- und 3-Phenoxy-4,5-dichlor-1,2-methylendioxybenzol.

Die als Ausgangsprodukte zur Herstellung der erfindungsgemäßen Produkte zu verwendenden Benzodioxole der Formel Ia sind teilweise bekannt.

Sie können auf bekannte Weise durch Umsetzung von teilweise bekannten Brenzkatechinderivaten der Formel (Ib)

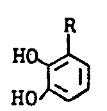

(Ib)

in welcher R die oben angegebene Bedeutung hat,

mit Dihalogenmethanen, wie z.B. Bromchlormethan, in Gegenwart eines Säureakzeptors und eines Verdünnungsmittels hergestellt werden.

Als Beispiele für die Ausgangsprodukte der Formel Ia seien genannt:

3-Methyl-, 3-Äthyl-, 3-n-Propyl-, 3-iso-Propyl-, 3-n-Butyl-3-iso-Butyl-, 3-sek.-Butyl- und 3-tert.-Butyl-, 3-Propen-(2)yl-, 3-(2-Methyl-propen(2)yl)-, 3-Buten(2)yl-, 3-Benzyl- und 3-Phenyl-1,2-methylendioxybenzol sowie 3-Methoxy-, 3-Äthoxy-, 3-n-Propoxy-, 3-iso-Propoxy-, 3-n-Butoxy-, 3-iso-Butoxy-, 3-sek.-Butoxy-, 3-tert.-Butoxy-, 3-Propen(2)oxy-, 3-(2-Methyl-propen(2)oxy)-, 3-Buten(2)oxy-, 3-Benzyloxy- und 3-Phenoxy-1,2-methylendioxybenzol.

Das Verfahren zur Herstellung der erfindungsgemäßen Verbindungen wird gegebenenfalls unter Verwendung geeigneter Verdünnungsmittel durchgeführt. Als solche kommen praktisch alle inerten organischen Solventien infrage. Hierzu gehören insbesondere aliphatische und aromatische, gegebenenfalls chlorierte Kohlenwasserstoffe, wie Benzol, Toluol, Xylol, Methylenchlorid, Chloroform, Tetrachlorkohlenstoff, Chlorbenzol und Dichlorbenzol.

Die Reaktionstemperaturen können in einem größeren Bereich variiert werden. Im allgemeinen arbeitet man zwischen 0 und 100°C, vorzugsweise zwischen 20 und 80°C. Die Umsetzung wird im allgemeinen bei Normaldruck durchgeführt.

Zur Durchführung des Verfahrens setzt man im allgemeinen je Mol Benzodioxolderivat zwischen 2 und 4 Mol, vorzugsweise zwischen 2,2 und 3 Mol Sulfurylchlorid ein. Im allgemeinen werden die beiden Reaktionspartner in einem der angegebenen Verdünnungsmittel bei den angegebenen Temperaturen eingesetzt.

Die Aufarbeitung erfolgt durch Waschen der Reaktionsmischung mit Wasser, Trocknen der organischen Phase und Abziehen des Lösungsmittels im Vakuum. Das im Rückstand verbleibende Rohprodukt wird durch Vakuumdestillation bzw. durch Umkristallisation gereinigt.

Zur Charakterisierung der Produkte dient der Brechungsindex oder der Siedepunkt bzw. der Schmelzpunkt.

Die Wirkstoffkombinationen eignen sich bei guter Pflanzenverträglichkeit und günstiger Warmblütertoxizität zur Bekämpfung von tierischen Schädlingen, insbesondere Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam.

Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophaus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips fermoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Chloristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium

pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Die Wirkstoffkombinationen können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate, sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoffkombination, vorzugsweise zwischen 0,5 und 90%, wobei die in der Wirkstoffkombination das Gewichtsverhältnis von Benzodioxolderivaten zu Wirkstoffen vorzugsweise zwischen 0,1 : 10 und 10 : 0,1 liegt.

Die Anwendung der erfindungsgemäßen Wirkstoffkombinationen erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffkombinationen durch eine hervorragende Residualwirkung auf Holz und Ton sowie durch eine gute Alkalistabilität auf gekälkten Unterlagen aus.

## Beispiel A

$LT_{100}$-Test

Testtiere: Phosphorsäureesterresistente Musca domestica (Stamm Weymanns)

Lösungsmittel: Aceton

Von den Wirkstoffen, Synergisten und Gemischen aus Wirkstoffen und Synergisten werden Lösungen hergestellt und 2,5 ml davon in Petrischalen auf Filterpapierscheiben von 9,5 cm Durchmesser pipetiert. Das Filterpapier saugt die Lösungen auf. Die Petrischalen bleiben so lange offen stehen, bis

das Lösungsmittel vollständig verdunstet ist. Anschließend gibt man 25 Testtiere in die Petrischalen und bedeckt sie mit einem Glasdeckel.

Der Zustand der Testtiere wird bis zu 6 Stunden laufend kontrolliert. Es wird diejenige Zeit ermittelt, die für eine 100%ige knock down-Wirkung erforderlich ist. Wird die $LT_{100}$ nach 6 Stunden nicht erreicht, wird der % Satz der knock down gegangenen Testtiere festgestellt.

Bei diesem Test zeigen z.B. die folgenden Verbindungen der Herstellungsbeispiele als Synergisten überlegene Wirkung gegenüber dem Stand der Technik: 1, 2, 3, 4, 5.

Herstellungsbeispiele

Beispiel 1

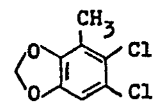

21,8 g (0,16 Mol) 3-Methyl-1,2-methylendioxybenzol und 45,7 g (0,384 Mol) Sulfurylchlorid werden in 200 ml Methylenchlorid zwei Stunden unter Rückfluß zum Sieden erhitzt und gerührt. Dann wird die Lösung abgekühlt und nacheinander mit Wasser und Natriumhydrogencarbonatlösung gewaschen. Die organische Phase wird getrocknet, das Lösungsmittel abgezogen und der Rückstand durch Verreiben mit Äther/Petroläther (2:1) zur Kristallisation gebracht.

Ausbeute 21 g (64% der Theorie), Schmelzpunkt 102°C.

Beispiel 2

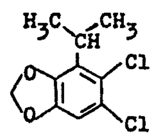

32,8 g (0,2 Mol) 3-iso-Propyl-1,2-methylendioxybenzol und 57 g (0,48 Mol) Sulfurylchlorid werden in 250 ml Methylenchlorid zwei Stunden unter Rückfluß zum Sieden erhitzt und gerührt. Nach dem Abkühlen wird die Lösung mit Wasser neutral gewaschen und getrocknet. Das Lösungsmittel wird abgezogen und das zurückbleibende Rohprodukt durch Vakuumdestillation gereinigt.

Ausbeute 39,6 g (85% der Theorie), Siedepunkt 138°C/5 Torr.

Analog Beispiel 1 oder 2 können folgende Verbindungen hergestellt werden:

| Beispiel Nr. | R | Ausbeute (% der Theorie) | Brechungsindex ($n_D^{20}$) bzw. Schmelzpunkt (°C) |
|---|---|---|---|
| 3 | iso-Butyl | 92 | 1,5438 |
| 4 | Methoxy | 64 | 80 |
| 5 | Äthoxy | 47 | 74 |

**Patentansprüche**

1. Dichlorbenzodioxolderivate der Formel (I)

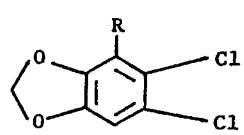

(I)

in welcher

R für geradkettiges oder verzweigtes Alkyl mit 1 bis 6, insbesondere 1 bis 4 Kohlenstoffatomen je Alkylrest, für geradkettiges oder verzweigtes Alkenyl mit 2 bis 6, insbesondere 2 bis 4 Kohlenstoffatomen je Alkenylrest,
für Benzyl und Phenyl,
für geradkettiges oder verzweigtes O-Alkyl mit 1 bis 6, insbesondere 1 bis 4 Kohlenstoffatomen je

Alkylrest, für geradkettiges oder verzweigtes O-Alkenyl mit 2 bis 6, insbesondere 2 bis 4 Kohlenstoffatomen je Alkenylrest, für Benzyloxy oder Phenoxy steht.

2. Verfahren zur Herstellung der Dichlorbenzodioxolderivate der Formel I gemäß Anspruch 1, dadurch gekennzeichnet, daß man Benzodioxolderivate der Formel (Ia)

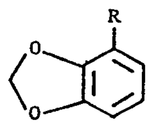

(Ia)

in welcher
R die oben angegebene Bedeutung hat,
mit Sulfurylchlorid, gegebenenfalls in Gegenwart eines Verdünnungsmittels, umsetzt.

3. Insektizide Mittel, gekennzeichnet durch einen Gehalt an einer Wirkstoffkombination bestehend aus Dichlorbenzodioxolderivaten der allgemeinen Formel (I) gemäß Anspruch 1
und

A) Carbamaten und/oder
B) Carbonsäureestern, einschließliche der natürlichen sowie synthetischen Pyrethroide, und/oder
C) Phosphorsäureestern und/oder
D) Halogenalkanen.

4. Insektizide Mittel gemäß Anspruch 3, dadurch gekennzeichnet, daß in der Wirkstoffkombination das Gewichtsverhältnis von Benzodioxolderivaten zu Wirkstoffen zwischen 0,1 : 10 und 10 : 0,1 liegt.

5. Verwendung von Wirkstoffkombinationen gemäß Anspruch 3 oder 4 zur Bekämpfung von Insekten.

6. Verfahren zur Herstellung von insektiziden Mitteln, dadurch gekennzeichnet, daß man eine Wirkstoffkombination gemäß Anspruch 3 oder 4 mit Streckmitteln und/oder oberflächenaktiven Stoffen vermischt.

**Revendications**

1. Dérivés de dichlorobenzodioxoles de formule (I):

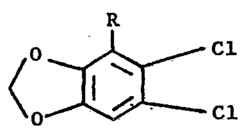

(I)

dans laquelle
R représente un groupe alkyle à chaîne droite ou ramifiée contenant 1 à 6 atomes de carbone, en particulier, 1 à 4 atomes de carbone dans chaque fraction alkyle, un groupe alcényle à chaîne droite ou ramifiée contenant 2 à 6 atomes de carbone, en particulier, 2 à 4 atomes de carbone dans chaque fraction alcényle,
un groupe benzyle et un groupe phényle,
un groupe O-alkyle à chaîne droite ou ramifiée contenant 1 à 6 atomes de carbone, en particulier, 1 à 4 atomes de carbone dans chaque fraction alkyle,
un groupe O-alcényle à chaîne droite ou ramifiée contenant 2 à 6 atomes de carbone, en particulier, 2 à 4 atomes de carbone dans chaque fraction alcényle,
un groupe benzyloxy ou un groupe phénoxy.

2. Procédé de préparation de dérivés de dichlorobenzodioxoles de formule I suivant la revendication 1, caractérisé en ce qu'on fait réagir des dérivés de benzodioxoles de formule (Ia):

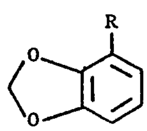

(Ia)

dans laquelle

R a la signification indiquée ci-dessus, avec du chlorure de sulfuryle, éventuellement en présence d'un diluant.

3. Agents insecticides, caractérisés en ce qu'ils contiennent une combinaison de substances actives comprenant des dérivés de dichlorobenzodioxoles de formule générale (I) suivant la revendication 1
et

A) des carbamates et/ou
B) des esters d'acides carboxyliques, y compris les pyréthroïdes naturels et synthétiques, et/ou
C) des esters de l'acide phosphorique et/ou
D) des halogénoalcanes.

4. Agents insecticides suivant la revendication 3, caractérisés en ce que, dans le combinaison de substances actives, le rapport pondéral entre les dérivés de benzodioxoles et les substances actives se situe entre 0,1:10 et 10:0,1.

5. Utilisation de combinaisons de substances actives suivant la revendication 3 ou 4 en vue de combattre les insectes.

6. Procédé de préparation d'agents insecticides, caractérisé en ce qu'on mélange une combinaison de substances actives suivant la revendication 3 ou 4 avec des diluants et/ou des substances tensio-actives.

**Claims**

1. Dichlorobenzodioxole derivatives of the formula (I)

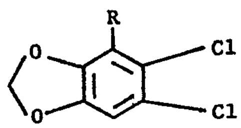

(I)

in which

R represents straight-chain or branched alkyl with 1 to 6, especially 1 to 4 carbon atoms per alkyl radical;
straight-chain or branched alkenyl with 2 to 6, especially 2 to 4 carbon atoms per alkenyl radical; benzyl and phenyl;
straight-chain or branched O-alkyl with 1 to 6, especially 1 to 4 carbon atoms per alkyl radical; straight-chain or branched O-alkenyl with 2 to 6, especially 2 to 4 carbon atoms per alkenyl radical; benzyloxy or phenoxy.

2. Process for the preparation of the dichlorobenzodioxole derivatives of the formula I according to Claim 1, characterised in that benzodioxole derivatives of the formula (Ia)

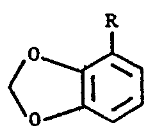

(Ia)

in which

R has the abovementioned meaning are reacted with sulphuryl chloride, if appropriate in the presence of a diluent.

3. Insecticidal agents, characterised in that they contain an active compound combination consisting of dichlorobenzodioxole derivatives of the general formula (I) according to Claim 1 and
A) carbamates and/or
B) carboxylic acid esters, including the natural and synthetic pyrethroids, and/or
C) phosphoric acid esters and/or
D) halogenoalkanes.

4. Insecticidal agents according to Claim 3, characterised in that in the active compound combination the weight ratio of benzodioxole derivatives to active compounds is between 0.1 : 10 and 10 : 0.1.

5. Use of active compound combinations according to Claim 3 or 4 for combating insects.

6. Process for the preparation of insecticidal agents, characterised in that an active compound combination according to Claim 3 or 4 is mixed with extenders and/or surface-active materials.